# EUROPEAN PATENT APPLICATION

(11) **EP 3 510 920 A1**
(43) Date of publication of application: **17.07.2019**
(21) Application number: 19158071.1
(22) Date of filing: 25.09.2014
(51) Int. Cl.: A61B 5/0402, A61B 5/11, A61B 5/055, A61B 5/00, A61B 5/0408, A61B 5/0428, H01M 2/10, H01M 4/74

(54) **EXTENDED WEAR ELECTROCARDIOGRAPHY PATCH USING INTERLACED WIRE ELECTRODES**

(30) Priority: 25.09.2013 US 201361882403 P; 14.11.2013 US 201314080717; 19.08.2014 US 201414463585; 19.08.2014 US 201414463571
(62) Divisional of application: 14781025.3
(71) Applicant: Bardy Diagnostics, Inc., Charlotte, NC 28207 (US)
(72) Inventor: FELIX, Jason, Vashon Island, WA Washington 98070 (US); BISHAY, Jon Mikalson, Lexington, KY Kentucky 40502 (US); BARDY, Gust H., Carnation, WA Washington 98014 (US)
(74) Representative: Hanna Moore + Curley

(57) **Abstract**

Physiological monitoring can be provided through a wearable monitor (12) that includes a flexible extended wear electrode patch (15) and a removable reusable monitor recorder (14). A pair of flexile wires (61, 71) is interlaced or sewn into a flexible backing (20), serving as electrode signal pickup and electrode circuit traces. The monitor (12) sits centrally on the patient's chest along the sternum (13), which significantly improves the ability to sense cutaneously cardiac electric signals, particularly those generated by the atrium. To counter the dislodgment due to compressional and torsional forces, non-irritating adhesive (43) is provided on the underside, or contact, surface of the electrode patch (15), but only on the distal (30) and proximal ends (31). Interlacing the flexile wires (61, 71) into the flexile backing (20) also provides structural support and malleability against compressional, tensile and torsional forces.

## Description

### TECHNICAL FIELD

This application relates in general to electrocardiographic monitoring and manufacture and, in particular, to an extended wear electrocardiography patch using interlaced wire electrodes.

### BACKGROUND ART

An electrocardiogram (ECG) is a tool used by physicians to diagnose heart problems and other potential health concerns. A full 12-lead ECG provides a multi-vector snapshot of heart function, typically recorded over 12 seconds, that can help diagnose rate and regularity of heartbeats, effect of drugs or cardiac devices, including pacemakers and implantable cardioverter-defibrillators (ICDs), and whether a patient has heart disease of any sort. Full 12-lead ECGs are used in-clinics or hospitals and, as a result, are limited to recording only those heart-related aspects present at the time of recording. Sporadic conditions that may not show up during a 12-second ECG recording require other means to diagnose them. These sporadic conditions include fainting or syncope; rhythm disorders, such as tachyarrhythmias and bradyarrhythmias; apneic episodes; and other cardiac and related disorders. Thus, a 12-lead ECG only provides a partial picture and can be insufficient for complete patient diagnosis of many cardiac disorders.

Diagnostic efficacy of problems, like syncope or cardiac arrhythmias, can be improved through the use of long-term extended wear ECG monitoring. Recording sufficient ECG and related physiological data over an extended period of time remains a significant challenge to healthcare providers, despite over a 40-year history of such efforts. Extended period monitoring essentially enables a physician to identify cardiac conditions, specifically, rhythm disorders, and other physiological events of potential concern. A 30-day observation day period is considered the "gold standard" of ECG monitoring, yet achieving a 30-day observation day period has heretofore proven unworkable because such ECG monitoring systems are arduous to employ, cumbersome to the patient, and excessively costly to manufacture and deploy. Nevertheless, if a patient's ECG could be recorded in an ambulatory setting over prolonged time periods, thereby allowing the patient to engage in activities of daily living, the chances of acquiring meaningful medical information and capturing an abnormal event while the patient is engaged in normal activities becomes more likely to be achieved.

Conventionally, maintaining continual contact between ECG electrodes and the skin after a day or two has been a problem. Time, dirt, moisture, and other environmental contaminants, as well as perspiration, skin oil, and dead skin cells from the patient's body, can get between an ECG electrode's non-conductive adhesive and the skin's surface. All of these factors adversely affect electrode adhesion and the quality of cardiac signal recordings. Furthermore, the physical movements of the patient and their clothing impart various compressional, tensile, and torsional forces on the contact point of an ECG electrode, especially over long recording times, and an inflexibly fastened ECG electrode will be prone to becoming dislodged. Moreover, dislodgment may occur unbeknownst to the patient, making the ECG recordings worthless. Further, some patients may have skin that is susceptible to itching or irritation, and the wearing of ECG electrodes can aggravate such skin conditions. Thus, a patient may want or need to periodically remove or replace ECG electrodes during a long-term ECG monitoring period, whether to replace a dislodged electrode, reestablish better adhesion, alleviate itching or irritation, allow for cleansing of the skin, allow for showering and exercise, or for other purpose. Such replacement or slight alteration in electrode location actually facilitates the goal of recording the ECG signal for long periods of time.

In addition, the high cost of the patient-wearable components used to provide long-term extended ECG monitoring can negatively influence the availability and use of monitors. Ideally, disposable, single-use components, such as adhesive electrodes, should be low cost, while other components of higher complexity, particularly the electronics hardware that detects and records ECG and related physiology, may be of unavoidably higher cost. To a degree, costs can be balanced by designing higher complexity components to be re-usable, but when the total cost of a full ECG monitoring ensemble remains high, despite the utilization of re-usable parts, the number of monitors available for use by healthcare providers can be inhibited. Cost, then, becomes a barrier to entry, which, in turn, can hinder or prevent healthcare providers from obtaining the means with which to efficaciously identify the physiology underlying sporadic cardiac arrhythmic conditions and can ultimately contribute to a failure to make a proper and timely medical diagnosis.

Conventionally, Holter monitors are widely used for long-term extended ECG monitoring. Typically, they are often used for only 24-48 hours. A typical Holter monitor is a wearable and portable version of an ECG that include cables for each electrode placed on the skin and a separate battery-powered ECG recorder. The cable and electrode combination (or leads) are placed in the anterior thoracic region in a manner similar to what is done with an in-clinic standard ECG machine. The duration of a Holter monitoring recording depends on the sensing and storage capabilities of the monitor, as well as battery life. A "looping" Holter (or event) monitor can operate for a longer period of time by overwriting older ECG tracings, thence "recycling" storage in favor of extended operation, yet at the risk of losing event data. Although capable of extended ECG monitoring, Holter monitors are cumbersome, expensive and typically only available by medical prescription, which limits their usability. Further, the skill required to properly place the electrodes on the patient's chest hinders or precludes a patient from replacing or removing the precordial leads and usually involves moving the patient from the physician office to a specialized center within the hospital or clinic.

The ZIO XT Patch and ZIO Event Card devices, manufactured by iRhythm Tech., Inc., San Francisco, CA, are wearable stick-on monitoring devices that are typically worn on the upper left pectoral region to respectively provide continuous and looping ECG recording. The location is used to simulate surgically implanted monitors. Both of these devices are prescription-only and for single patient use. The ZIO XT Patch device is limited to a 14-day monitoring period, while the electrodes only of the ZIO Event Card device can be worn for up to 30 days. The ZIO XT Patch device combines both electronic recordation components and physical electrodes into a unitary assembly that adheres to the patient's skin. The ZIO XT Patch device uses adhesive sufficiently strong to support the weight of both the monitor and the electrodes over an extended period of time and to resist disadherence from the patient's body, albeit at the cost of disallowing removal or relocation during the monitoring period. The ZIO Event Card device is a form of downsized Holter monitor with a recorder component that must be removed temporarily during baths or other activities that could damage the non-waterproof electronics. Both devices represent compromises between length of wear and quality of ECG monitoring, especially with respect to ease of long term use, female-friendly fit, and quality of cardiac electrical potential signals, especially atrial (P-wave) signals.

Therefore, a need remains for a low cost extended wear continuously recording ECG monitor practicably capable of being worn for a long period of time, especially in patient's whose breast anatomy can interfere with signal quality in both men and women and that is capable of recording atrial action potential signals reliably.

### DISCLOSURE OF THE INVENTION

Physiological monitoring can be provided through a lightweight wearable monitor that includes two components, a flexible extended wear electrode patch and a reusable monitor recorder that removably snaps into a receptacle on the electrode patch. The wearable monitor sits centrally (in the midline) on the patient's chest along the sternum oriented top-to-bottom. The placement of the wearable monitor in a location at the sternal midline (or immediately to either side of the sternum), with its unique narrow "hourglass"-like shape, significantly improves the ability of the wearable monitor to cutaneously sense cardiac electrical potential signals, particularly the P-wave (or atrial activity) and, to a lesser extent, the QRS interval signals indicating ventricular activity in the ECG waveforms.

The electrode patch is shaped to fit comfortably and conformal to the contours of the patient's chest approximately centered on the sternal midline. To counter the dislodgment due to compressional and torsional forces, a layer of non-irritating adhesive, such as hydrocolloid, is provided at least partially on the underside, or contact, surface of the electrode patch, but only on the electrode patch's distal and proximal ends, where the electrode signal pickups are located. The unadhesed narrowed midsection rides freely over the skin. To counter dislodgment due to tensile and torsional forces, a flexible backing is reinforced with a flexile wire interlaced longitudinally through the narrowed midsection, with the curvature of the flexile wire providing both structural support and malleability. Each of these components are distinctive and allow for comfortable and extended wear, especially for women, where breast mobility would otherwise interfere with monitor use and wearer comfort.

Moreover, the interlacing of flexile wire simplifies manufacturing and reduces costs. A simple pair of flexile wires are used, instead of custom point-to-point circuit traces, to connect each electrode signal pickup to the receptacle. One end of each flexile wire can be sewn into the receptacle's circuit board, thereby obviating the need for conductive adhesive, soldered or electromechanical connection, and the other end of each flexile wire, when stripped of insulation, can act as an electrode signal pickup, which lowers component count.

One embodiment provides an extended wear electrocardiography patch using interlaced wire electrodes. A flexible backing is formed of an elongated strip of stretchable material with a narrow longitudinal midsection evenly tapering inward from a distal end and a proximal end, the elongated strip adherable only to a contact surface defined on each of the ends. A pair of flexile wires make up a pair of electrodes, one such electrode comprising one of the flexile wires interlaced into the distal end of the elongated strip and continuing back along an axial path through the narrow longitudinal midsection, another such electrode comprising the other of the flexile wires interlaced into the proximal end of the elongated strip, each of the electrodes further comprising an electrically conductive area only exposed on the contact surface. A circuit board is affixed on the proximal end of the elongated strip, comprising a battery compartment operable to hold a battery for powering an electrocardiography monitor, and an electrical pad operable to electrically couple the electrocardiography monitor with the pair of the electrodes. Alternatively, the circuit board is replaced by a housing that is affixed on the proximal end of the elongated strip, comprising a battery compartment operable to hold a battery for powering an electrocardiography monitor and a plurality of electrical pads operable to electrically couple the electrocardiography monitor with the pair of the electrodes. The housing can be affixed to the proximal end through the interlacing of the flexile wire. A non-conductive receptacle is securely adhered on the one end of the elongated strip opposite the contact surface and formed to removably receive the electrocardiography monitor, the non-conductive receptacle comprising electrode terminals aligned to electrically interface the pair of the flexile wires to the electrocardiography monitor.

A further embodiment provides an extended wear electrocardiography patch using non-metal electrodes. A flexible backing is formed of an elongated strip of stretchable material with a narrow longitudinal midsection evenly tapering inward from a distal end and a proximal end, the elongated strip adherable only to a contact surface defined on each of the ends. A pair of flexile wires makes up a pair of electrodes, one such electrode comprising one of the flexile wires positioned on the distal end of the elongated strip on the contact surface and continuing back along an axial path through the narrow longitudinal midsection, another such electrode comprising the other of the flexile wires positioned on the proximal end of the elongated strip on the contact surface, each of the electrodes being embedded in an electrically conductive non-metal adhesive. A circuit board is affixed on the proximal end of the elongated strip, comprising a battery compartment operable to hold a battery for powering an electrocardiography monitor, and an electrical pad operable to electrically couple the electrocardiography monitor with the pair of the electrodes. Alternatively, the circuit board is replaced by a housing that is affixed on the proximal end of the elongated strip, comprising a battery compartment operable to hold a battery for powering an electrocardiography monitor and a plurality of electrical pads operable to electrically couple the electrocardiography monitor with the pair of the electrodes. The housing can be affixed to the proximal end through the interlacing of the flexile wires. A non-conductive receptacle is securely adhered on the one end of the elongated strip opposite the contact surface and formed to removably receive the electrocardiography monitor, the non-conductive receptacle includes electrode terminals aligned to electrically interface the pair of the flexile wires to the electrocardiography monitor.

A still further embodiment provides a method for constructing a stress-pliant physiological electrode assembly. An electrode backing is formed from a stretchable woven textile material compatible to contact the skin on at least one surface. A length of flexile wire is obtained, the flexile wire having a cross-sectional girth greater than the interstices formed between individual threads comprised in the stretchable woven textile material. An electrical contact area on the at least one surface area is defined, and an electrically-contacting end of the flexile wire is positioned substantially within the electrical contact area. The flexile wire is interlaced through the stretchable woven textile material from the electrical contact area to a surface of the stretchable woven textile material opposite to the electrical contact area and is operable to receive an electrical connection.

A still further embodiment provides an extended wear electrocardiography patch. A flexible backing is formed of an elongated strip of stretchable material with a narrow longitudinal midsection evenly tapering inward from both ends. The elongated strip is adherable only on each end of a contact surface to serve as a crimp relief to facilitate compression of the narrow longitudinal midsection in response to compressional and torsional forces. A pair of electrocardiographic electrodes is respectively affixed to and conductively exposed on the contact surface of each end of the elongated strip. A flexible circuit is affixed on each end to the elongated strip. The flexible circuit includes a pair of circuit traces both originating within one of the ends of the elongated strip and which are electrically coupled to each electrocardiographic electrode. A laterally-extendable strain relief is defined in the flexible circuit and formed to facilitate extension and rotation of the flexible circuit in response to tensile and torsional forces. A non-conductive receptacle is securely adhered on the one end of the elongated strip opposite the contact surface and is formed to removably receive an electrocardiography monitor. The non-conductive receptacle includes electrode terminals aligned to electrically interface the pair of circuit traces to the electrocardiography monitor.

A still further embodiment provides an extended wear hourglass-shaped electrocardiography patch. A flexible backing is formed of an elongated strip of stretchable material with a narrow longitudinal midsection evenly tapering inward from both ends into an hourglass-shaped pattern when viewed from above. A contact surface of the flexible backing includes a non-irritating adhesive dressing at least partially coated on each end and lacking the non-irritating adhesive dressing on the narrow longitudinal midsection. A pair of electrocardiographic electrodes is respectively affixed to and conductively exposed on the contact surface of each end of the flexible backing. A flexible circuit is affixed to each end to the flexible backing. A pair of circuit traces both originate within one of the ends of the flexible backing. Each circuit trace is electrically coupled to one of the electrocardiographic electrodes. A pair of strain relief cutouts each face towards the other strain relief cutout. Each strain relief cutout partially extends transversely from and continuing longitudinally to a side of the flexible circuit different than the other strain relief cutout. One of the pair of circuit traces follows a path formed on the flexible circuit between the pair of strain relief cutouts. A non-conductive receptacle is securely adhered on the one end of the flexible backing opposite the contact surface and is formed to removably receive an electrocardiography monitor.

The monitoring patch is especially suited to the female anatomy, although also easily used over the male sternum. The narrow longitudinal midsection can fit nicely within the intermammary cleft of the breasts without inducing discomfort, whereas conventional patch electrodes are wide and, if adhered between the breasts, would cause chafing, irritation, discomfort, and annoyance, leading to low patient compliance.

The foregoing aspects enhance ECG monitoring performance and quality by facilitating long-term ECG recording, which is critical to accurate arrhythmia and cardiac rhythm disorder diagnoses.

In addition, the foregoing aspects enhance comfort in women (and certain men), but not irritation of the breasts, by placing the monitoring patch in the best location possible for optimizing the recording of cardiac signals from the atrium, particularly P-waves, which is another feature critical to proper arrhythmia and cardiac rhythm disorder diagnoses.

Further, the interlaced flexile wires improve the dermal electrode's response to tensile, twisting, compressional, and torsional forces by providing a strain relief and tensile strength, while also diminish the cost and complexity of producing physiological electrode assemblies and other types of electrical circuits, where point-to-point interconnections are needed.

Still other embodiments will become readily apparent to those skilled in the art from the following detailed description, wherein are described embodiments by way of illustrating the best mode contemplated. As will be realized, other and different embodiments are possible and the embodiments' several details are capable of modifications in various obvious respects, all without departing from their spirit and the scope. Accordingly, the drawings and detailed description are to be regarded as illustrative in nature and not as restrictive.

### DESCRIPTION OF THE DRAWINGS

FIGURES 1 and 2 are diagrams showing, by way of examples, an extended wear electrocardiography monitor, including an extended wear electrode patch in accordance with one embodiment, respectively fitted to the sternal region of a female patient and a male patient.
FIGURE 3 is a perspective view showing an extended wear electrode patch in accordance with one embodiment with a monitor recorder inserted.
FIGURE 4 is a perspective view showing the extended wear electrode patch of FIGURE 3 without a monitor recorder inserted.
FIGURE 5 is a top view showing the flexible circuit of the extended wear electrode patch of FIGURE 3.
FIGURE 6 is a perspective view showing the extended wear electrode patch in accordance with a further embodiment.
FIGURE 7 is an exploded view showing the component layers of the electrode patch of FIGURE 3.
FIGURE 8 is a bottom plan view of the extended wear electrode patch of FIGURE 3 with liner partially peeled back.
FIGURE 9 is a perspective view of an extended wear electrode patch with a flexile wire electrode assembly in accordance with a still further embodiment.
FIGURE 10 is perspective view of the flexile wire electrode assembly from FIGURE 9, with a layer of insulating material shielding a bare distal wire around the midsection of the flexible backing.
FIGURE 11 is a bottom view of the flexile wire electrode assembly as shown in FIGURE 9.
FIGURE 12 is a bottom view of a flexile wire electrode assembly in accordance with a still yet further embodiment.
FIGURE 13 is a perspective view showing the longitudinal midsection of the flexible backing of the electrode assembly from FIGURE 9.
FIGURE 14 is a longitudinal cross-sectional view of the midsection of the flexible backing of the electrode assembly of FIGURE 11.
FIGURES 15A-C are the electrode assembly from FIGURE 14 under compressional, tensile, and bending force, respectively.
FIGURE 16 is a flow diagram showing a method for constructing a stress-pliant physiological electrode assembly in accordance with a further embodiment.

### BEST MODE FOR CARRYING OUT THE INVENTION

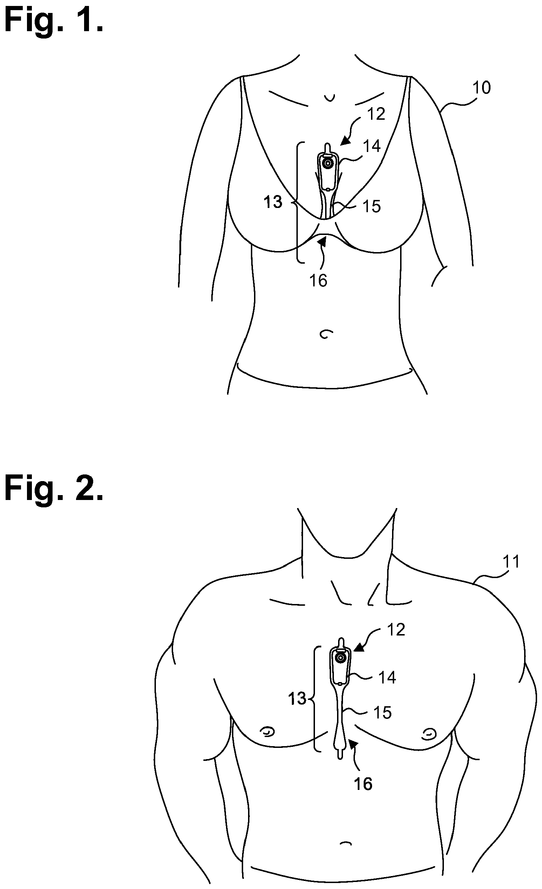

Physiological monitoring can be provided through a wearable monitor that includes two components, a flexible extended wear electrode patch and a removable reusable monitor recorder. FIGURES 1 and 2 are diagrams showing, by way of examples, an extended wear electrocardiography monitor 12, including an extended wear electrode patch 15 in accordance with one embodiment, respectively fitted to the sternal region of a female patient 10 and a male patient 11. The wearable monitor 12 sits centrally (in the midline) on the patient's chest along the sternum 13 oriented top-to-bottom with the monitor recorder 14 preferably situated towards the patient's head. The electrode patch 15 is shaped to fit comfortably and conformal to the contours of the patient's chest approximately centered on the sternal midline 16 (or immediately to either side of the sternum 13). The distal end of the electrode patch 15 extends towards the Xiphoid process and lower sternum and, depending upon the patient's build, may straddle the region over the Xiphoid process and lower sternum. The proximal end of the electrode patch 15, located under the monitor recorder 14, is below the manubrium and, depending upon patient's build, may straddle the region over the manubrium.

The placement of the wearable monitor 12 in a location at the sternal midline 16 (or immediately to either side of the sternum 13) significantly improves the ability of the wearable monitor 12 to cutaneously sense cardiac electric signals, particularly the P-wave (or atrial activity) and, to a lesser extent, the QRS interval signals in the ECG waveforms that indicate ventricular activity. The sternum 13 overlies the right atrium of the heart and the placement of the wearable monitor 12 in the region of the sternal midline 13 puts the ECG electrodes of the electrode patch 15 in a location better adapted to sensing and recording P-wave signals than other placement locations, say, the upper left pectoral region. In addition, placing the lower or inferior pole (ECG electrode) of the electrode patch 15 over (or near) the Xiphoid process and lower sternum facilitates sensing of right ventricular activity and provides superior recordation of the QRS interval.

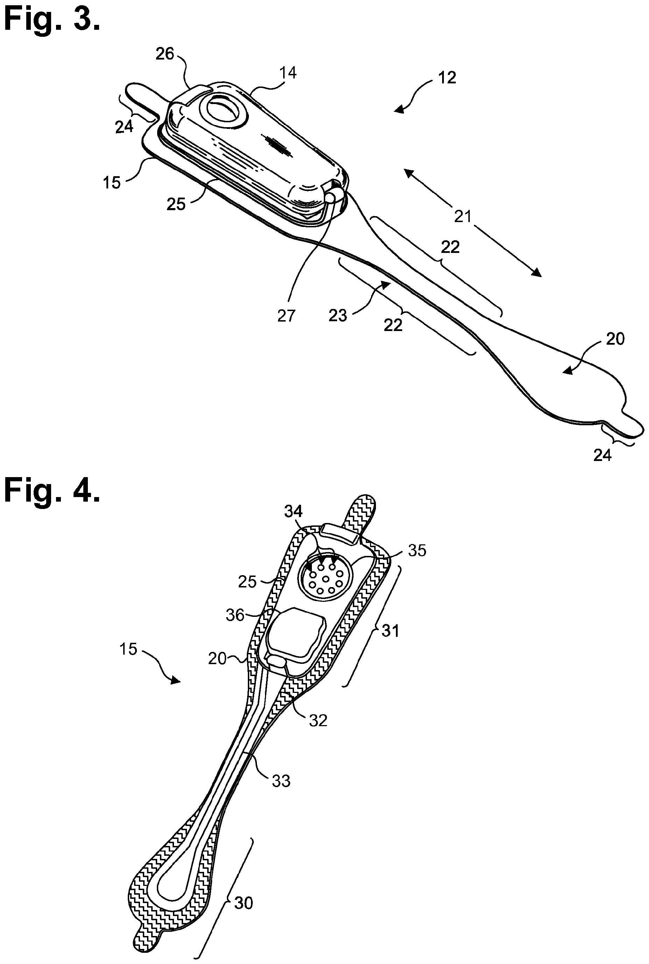

During use, the electrode patch 15 is first adhered to the skin along the sternal midline 16 (or immediately to either side of the sternum 13). A monitor recorder 14 is then snapped into place on the electrode patch 15 to initiate ECG monitoring. FIGURE 3 is a perspective view showing an extended wear electrode patch 15 in accordance with one embodiment with a monitor recorder 14 inserted. The body of the electrode patch 15 is preferably constructed using a flexible backing 20 formed as an elongated strip 21 of wrap knit or similar stretchable material about 145mm long and 32mm at the widest point with a narrow longitudinal mid-section 23 evenly tapering inward from both sides. A pair of cut-outs 22 between the distal and proximal ends of the electrode patch 15 create a narrow longitudinal midsection 23 or "isthmus" and defines an elongated "hourglass"-like shape, when viewed from above, such as described in commonly-assigned U.S. Design Patent application, entitled "Extended Wear Electrode Patch," Serial No. 29/472,045, filed November 7, 2013, pending, the disclosure of which is incorporated by reference. The upper part of the "hourglass" is sized to allow an electrically non-conductive receptacle 25, sits on top of the outward-facing surface of the electrode patch 15, to be affixed to the electrode patch 15 with an ECG electrode placed underneath on the patient-facing underside, or contact, surface of the electrode patch 15; the upper part of the "hourglass" has a longer and wider profile than the lower part of the "hourglass," which is sized primarily to allow just the placement of an ECG electrode.

The electrode patch 15 incorporates features that significantly improve wearability, performance, and patient comfort throughout an extended monitoring period. The entire electrode patch 15 is lightweight in construction, which allows the patch to be resilient to disadhesing or falling off and, critically, to avoid creating distracting discomfort to the patient, even when the patient is asleep. In contrast, the weight of a heavy ECG monitor impedes patient mobility and will cause the monitor to constantly tug downwards and press on the patient's body; frequent adjustments by the patient are needed to maintain comfort.

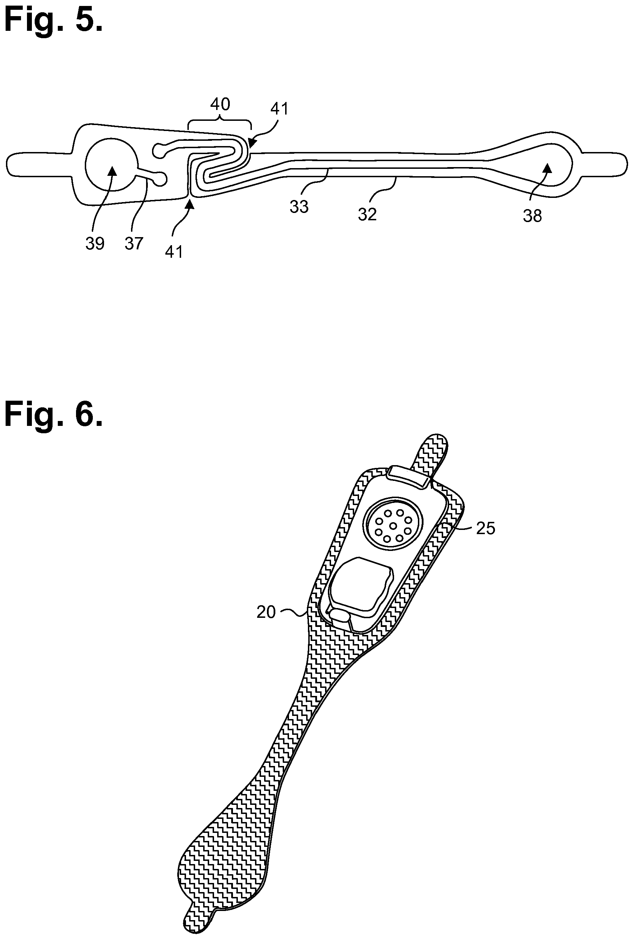

During every day wear, the electrode patch 15 is subjected to pushing, pulling, and torsional movements, including compressional and torsional forces when the patient bends forward, and tensile and torsional forces when the patient leans backwards. To counter these stress forces, the electrode patch 15 incorporates crimp and strain reliefs, as further described *infra* respectively with reference to FIGURES 4 and 5. In addition, the cut-outs 22 and longitudinal midsection 23 help minimize interference with and discomfort to breast tissue, particularly in women (and gynecomastic men). The cut-outs 22 and longitudinal midsection 23 allow better conformity of the electrode patch 15 to sternal bowing and to the narrow isthmus of flat skin that can occur along the bottom of the intermammary cleft between the breasts, especially in buxom women. The cut-outs 22 and longitudinal midsection 23 help the electrode patch 15 fit nicely between a pair of female breasts in the intermammary cleft. In one embodiment, the cut-outs 22 can be graduated to form the longitudinal midsection 23 as a narrow in-between stem or isthmus portion about 7mm wide. In a still further embodiment, tabs 24 can respectively extend an additional 8mm to 12mm beyond the distal and proximal ends of the flexible backing 20 to facilitate purchase when adhering the electrode patch 15 to or removing the electrode patch 15 from the sternum 13. These tabs preferably lack adhesive on the underside, or contact, surface of the electrode patch 15. Still other shapes, cut-outs and conformities to the electrode patch 15 are possible.

The monitor recorder 14 removably and reusably snaps into an electrically non-conductive receptacle 25 during use. The monitor recorder 14 contains electronic circuitry for recording and storing the patient's electrocardiography as sensed via a pair of ECG electrodes provided on the electrode patch 15, such as described in commonly-assigned U.S. Patent application, entitled "Extended Wear Ambulatory Electrocardiography and Physiological Sensor Monitor," Serial No. 14/080,725, filed November 14, 2013, pending, the disclosure of which is incorporated by reference. The circuitry includes a microcontroller, flash storage, ECG signal processing, analog-to-digital conversion (where applicable), and an external interface for coupling to the electrode patch 15 and to a download station for stored data download and device programming. The monitor recorder 14 also includes external patient-interfaceable controls, such as a push button to facilitate event marking and provide feedback. In a further embodiment, the circuitry, with the assistance of the appropriate types of deployed electrodes or sensors, is capable of monitoring other types of physiology, in addition to ECGs. Still other types of monitor recorder components and functionality are possible.

The non-conductive receptacle 25 is provided on the top surface of the flexible backing 20 with a retention catch 26 and tension clip 27 molded into the non-conductive receptacle 25 to conformably receive and securely hold the monitor recorder 14 in place. The edges of the bottom surface of the non-conductive receptacle 25 are preferably rounded, and the monitor recorder 14 is nestled inside the interior of the non-conductive receptacle 25 to present a rounded (gentle) surface, rather than a sharp edge at the skin-to-device interface.

The electrode patch 15 is intended to be disposable. The monitor recorder 14, however, is reusable and can be transferred to successive electrode patches 15 to ensure continuity of monitoring. The placement of the wearable monitor 12 in a location at the sternal midline 16 (or immediately to either side of the sternum 13) benefits long-term extended wear by removing the requirement that ECG electrodes be continually placed in the same spots on the skin throughout the monitoring period. Instead, the patient is free to place an electrode patch 15 anywhere within the general region of the sternum 13.

As a result, at any point during ECG monitoring, the patient's skin is able to recover from the wearing of an electrode patch 15, which increases patient comfort and satisfaction, while the monitor recorder 14 ensures ECG monitoring continuity with minimal effort. A monitor recorder 14 is merely unsnapped from a worn out electrode patch 15, the worn out electrode patch 15 is removed from the skin, a new electrode patch 15 is adhered to the skin, possibly in a new spot immediately adjacent to the earlier location, and the same monitor recorder 14 is snapped into the new electrode patch 15 to reinitiate and continue the ECG monitoring.

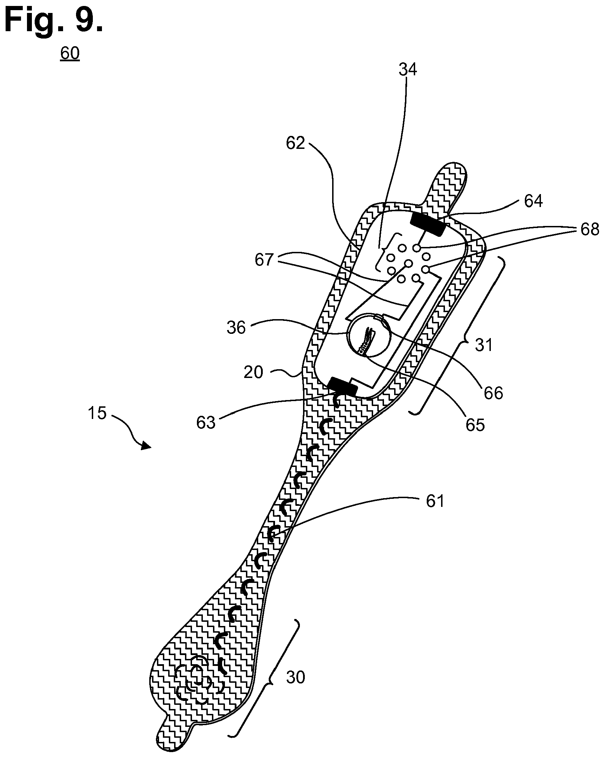

During use, the electrode patch 15 is first adhered to the skin in the sternal region. FIGURE 4 is a perspective view showing the extended wear electrode patch 15 of FIGURE 3 without a monitor recorder 14 inserted. A flexible circuit 32 is adhered to each end of the flexible backing 20. A distal circuit trace 33 from the distal end 30 of the flexible backing 20 and a proximal circuit trace (not shown) from the proximal end 31 of the flexible backing 20 electrically couple ECG electrodes (not shown) with a pair of electrical pads 34. In a further embodiment, the distal and proximal circuit traces are replaced with interlaced or sewn-in flexible wires, as further described *infra* beginning with reference to FIGURE 9. The electrical pads 34 are provided within a moisture-resistant seal 35 formed on the bottom surface of the non-conductive receptacle 25. When the monitor recorder 14 is securely received into the non-conductive receptacle 25, that is, snapped into place, the electrical pads 34 interface to electrical contacts (not shown) protruding from the bottom surface of the monitor recorder 14. The moisture-resistant seal 35 enables the monitor recorder 14 to be worn at all times, even during bathing or other activities that could expose the monitor recorder 14 to moisture or adverse conditions.

In addition, a battery compartment 36 is formed on the bottom surface of the non-conductive receptacle 25. A pair of battery leads (not shown) from the battery compartment 36 to another pair of the electrical pads 34 electrically interface the battery to the monitor recorder 14. The battery contained within the battery compartment 35 can be replaceable, rechargeable or disposable.

The monitor recorder 14 draws power externally from the battery provided in the non-conductive receptacle 25, thereby uniquely obviating the need for the monitor recorder 14 to carry a dedicated power source. The battery contained within the battery compartment 36 can be replaceable, rechargeable or disposable. In a further embodiment, the ECG sensing circuitry of the monitor recorder 14 can be supplemented with additional sensors, including an Sp02 sensor, a blood pressure sensor, a temperature sensor, respiratory rate sensor, a glucose sensor, an air flow sensor, and a volumetric pressure sensor, which can be incorporated directly into the monitor recorder 14 or onto the non-conductive receptacle 25.

The placement of the flexible backing 20 on the sternal midline 16 (or immediately to either side of the sternum 13) also helps to minimize the side-to-side movement of the wearable monitor 12 in the left- and right-handed directions during wear. However, the wearable monitor 12 is still susceptible to pushing, pulling, and torqueing movements, including compressional and torsional forces when the patient bends forward, and tensile and torsional forces when the patient leans backwards. To counter the dislodgment of the flexible backing 20 due to compressional and torsional forces, a layer of non-irritating adhesive, such as hydrocolloid, is provided at least partially on the underside, or contact, surface of the flexible backing 20, but only on the distal end 30 and the proximal end 31. As a result, the underside, or contact surface of the longitudinal midsection 23 does not have an adhesive layer and remains free to move relative to the skin. Thus, the longitudinal midsection 23 forms a crimp relief that respectively facilitates compression and twisting of the flexible backing 20 in response to compressional and torsional forces. Other forms of flexible backing crimp reliefs are possible.

Unlike the flexible backing 20, the flexible circuit 32 is only able to bend and cannot stretch in a planar direction. FIGURE 5 is a top view showing the flexible circuit 32 of the extended wear electrode patch 15 of FIGURE 3. A distal ECG electrode 38 and proximal ECG electrode 39 are respectively coupled to the distal and proximal ends of the flexible circuit 32 to serve as electrode signal pickups. The flexible circuit 32 preferably does not extend to the outside edges of the flexible backing 20, thereby avoiding gouging or discomforting the patient's skin during extended wear, such as when sleeping on the side. During wear, the ECG electrodes 38, 39 must remain in continual contact with the skin. A strain relief 40 is defined in the flexible circuit 32 at a location that is partially underneath the battery compartment 36 when the flexible circuit 32 is affixed to the flexible backing 20. The strain relief 40 is laterally extendable to counter dislodgment of the ECG electrodes 38, 39 due to tensile and torsional forces. A pair of strain relief cutouts 41 partially extend transversely from each opposite side of the flexible circuit 32 and continue longitudinally towards each other to define in 'S'-shaped pattern, when viewed from above. The strain relief respectively facilitates longitudinal extension and twisting of the flexible circuit 32 in response to tensile and torsional forces. Other forms of circuit board strain relief are possible.

The flexible circuit 32 can be provided either above or below the flexible backing 20. FIGURE 6 is a perspective view showing the extended wear electrode patch 15 in accordance with a further embodiment. The flexible circuit (not shown) is provided on the underside, or contact, surface of the flexible backing 20 and is electrically interfaced to the set of electrical pads 34 on the bottom surface of the non-conductive receptacle 25 through electrical contacts (not shown) pierced through the flexible backing 20.

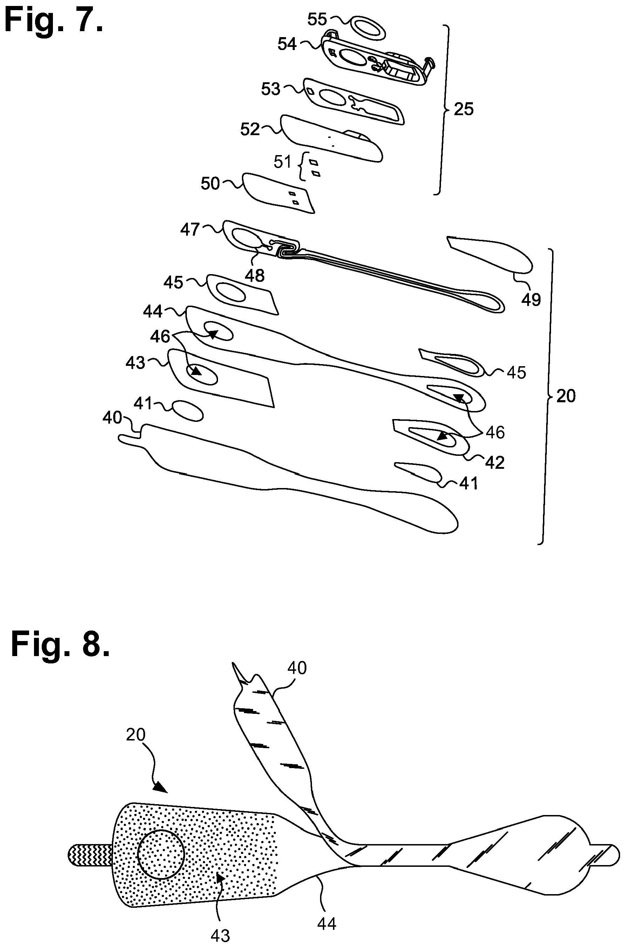

The electrode patch 15 is intended to be a disposable component, which enables a patient to replace the electrode patch 15 as needed throughout the monitoring period, while maintaining continuity of physiological sensing through reuse of the same monitor recorder 14. FIGURE 7 is an exploded view showing the component layers of the electrode patch 15 of FIGURE 3. The flexible backing 20 is constructed of a wearable gauze, latex, woven textile, or similar wrap knit or stretchable and wear-safe material 44, such as a Tricot-type linen with a pressure sensitive adhesive (PSA) on the underside, or contact, surface. The ends of the wearable material 44 are coated with a layer 43 of non-irritating adhesive, such as hydrocolloid, to facilitate long-term wear, while the unadhesed narrowed midsection rides freely over the skin. The hydrocolloid, for instance, is typically made of mineral oil, cellulose and water and lacks any chemical solvents, so should cause little itching or irritation. Moreover, hydrocolloid can be manufactured into an appropriate thickness and plasticity and provides cushioning between the relatively rigid and unyielding non-conductive receptacle 25 and the patient's skin. In a further embodiment, the layer of non-irritating adhesive can be contoured, such as by forming the adhesive with a concave or convex cross-section; surfaced, such as through stripes or crosshatches of adhesive, or by forming dimples in the adhesive's surface; or applied discontinuously, such as with a formation of discrete dots of adhesive.

As described *supra* with reference to FIGURE 5, a flexible circuit can be adhered to either the outward facing surface or the underside, or contact, surface of the flexible backing 20. For convenience, a flexible circuit 47 is shown relative to the outward facing surface of the wearable material 44 and is adhered respectively on a distal end by a distal electrode seal 45 and on a proximal end by a proximal electrode seal 45. In a further embodiment, the flexible circuit 47 can be provided on the underside, or contact, surface of the wearable material 44. Through the electrode seals, only the distal and proximal ends of the flexible circuit 47 are attached to the wearable material 44, which enables the strain relief 40 (shown in FIGURE 5) to respectively longitudinally extend and twist in response to tensile and torsional forces during wear. Similarly, the layer 43 of non-irritating adhesive is provided on the underside, or contact, surface of the wearable material 44 only on the proximal and distal ends, which enables the longitudinal midsection 23 (shown in FIGURE 3) to respectively bow outward and away from the sternum 13 or twist in response to compressional and torsional forces during wear.

A pair of openings 46 is defined on the distal and proximal ends of the wearable material 44 and layer 43 of non-irritating adhesive for ECG electrodes 38, 39 (shown in FIGURE 5). The openings 46 serve as "gel" wells with a layer of hydrogel 41 being used to fill the bottom of each opening 46 as a conductive material that aids electrode signal capture. The entire underside, or contact, surface of the flexible backing 20 is protected prior to use by a liner layer 40 that is peeled away, as shown in FIGURE 8.

The non-conductive receptacle 25 includes a main body 54 that is molded out of polycarbonate, ABS, or an alloy of those two materials to provide a high surface energy to facilitate adhesion of an adhesive seal 53. The main body 54 is attached to a battery printed circuit board 52 by the adhesive seal 53 and, in turn, the battery printed circuit board 52 is adhered to the flexible circuit 47 with an upper flexible circuit seal 50. A pair of conductive transfer adhesive points 51 or, alternatively, soldered connections, or electromechanical connections, including metallic rivets or similar conductive and structurally unifying components, connect the circuit traces 33, 37 (shown in FIGURE 5) of the flexible circuit 47 to the battery printed circuit board 52. The main body 54 has a retention catch 26 and tension clip 27 (shown in FIGURE 3) that fixably and securely receive a monitor recorder 14 (not shown), and includes a recess within which to circumferentially receive a die cut gasket 55, either rubber, urethane foam, or similar suitable material, to provide a moisture resistant seal to the set of pads 34. Other types of design, arrangement, and permutation are possible.

In a still further embodiment, the flexible circuit 32 (shown in FIGURE 4) and distal ECG electrode 38 and proximal ECG electrode 39 (shown in FIGURE 5) are replaced with a pair of interlaced flexile wires. The interlacing of flexile wires through the flexible backing 20 reduces both manufacturing costs and environmental impact, as further described *infra.* The flexible circuit and ECG electrodes are replaced with a pair of flexile wires that serve as both electrode circuit traces and electrode signal pickups. FIGURE 9 is a perspective view of an extended wear electrode patch 15 with a flexile wire electrode assembly in accordance with a still further embodiment. The flexible backing 20 maintains the unique narrow "hourglass"-like shape that aids long term extended wear, particularly in women, as described *supra* with reference to FIGURE 3. For clarity, the non-conductive receptacle 25 is omitted to show the exposed battery printed circuit board 62 that is adhered underneath the non-conductive receptacle 25 to the proximal end 31 of the flexible backing 20. Instead of employing flexible circuits, a pair of flexile wires are separately interlaced or sewn into the flexible backing 20 to serve as circuit connections for an anode electrode lead and for a cathode electrode lead.

To form a distal electrode assembly, a distal wire 61 is interlaced into the distal end 30 of the flexible backing 20, continues along an axial path through the narrow longitudinal midsection of the elongated strip, and electrically connects to the battery printed circuit board 62 on the proximal end 31 of the flexible backing 20. The distal wire 61 is connected to the battery printed circuit board 62 by stripping the distal wire 61 of insulation, if applicable, and interlacing or sewing the uninsulated end of the distal wire 61 directly into an exposed circuit trace 63. The distal wire-to-battery printed circuit board connection can be made, for instance, by back stitching the distal wire 61 back and forth across the edge of the battery printed circuit board 62. Similarly, to form a proximal electrode assembly, a proximal wire (not shown) is interlaced into the proximal end 31 of the flexible backing 20. The proximal wire is connected to the battery printed circuit board 62 by stripping the proximal wire of insulation, if applicable, and interlacing or sewing the uninsulated end of the proximal wire directly into an exposed circuit trace 64. The resulting flexile wire connections both establish electrical connections and help to affix the battery printed circuit board 62 to the flexible backing 20.

The battery printed circuit board 62 is provided with a battery compartment 36. A set of electrical pads 34 are formed on the battery printed circuit board 62. The electrical pads 34 electrically interface the battery printed circuit board 62 with a monitor recorder 14 when fitted into the non-conductive receptacle 25. The battery compartment 36 contains a spring 65 and a clasp 66, or similar assembly, to hold a battery (not shown) in place and electrically interfaces the battery to the electrical pads 34 through a pair battery leads 67 for powering the electrocardiography monitor 14. Other types of battery compartment are possible. The battery contained within the battery compartment 36 can be replaceable, rechargeable, or disposable.

In a yet further embodiment, the circuit board and non-conductive receptacle 25 are replaced by a combined housing that includes a battery compartment and a plurality of electrical pads. The housing can be affixed to the proximal end of the elongated strip through the interlacing or sewing of the flexile wires or other wires or threads.

The core of the flexile wires may be made from a solid, stranded, or braided conductive metal or metal compounds. In general, a solid wire will be less flexible than a stranded wire with the same total cross-sectional area, but will provide more mechanical rigidity than the stranded wire. The conductive core may be copper, aluminum, silver, or other material. The pair of the flexile wires may be provided as insulated wire. In one embodiment, the flexile wires are made from a magnet wire from Belden Cable, catalogue number 8051, with a solid core of AWG 22 with bare copper as conductor material and insulated by polyurethane or nylon. Still other types of flexile wires are possible. In a further embodiment, conductive ink or graphene can be used to print electrical connections, either in combination with or in place of the flexile wires.

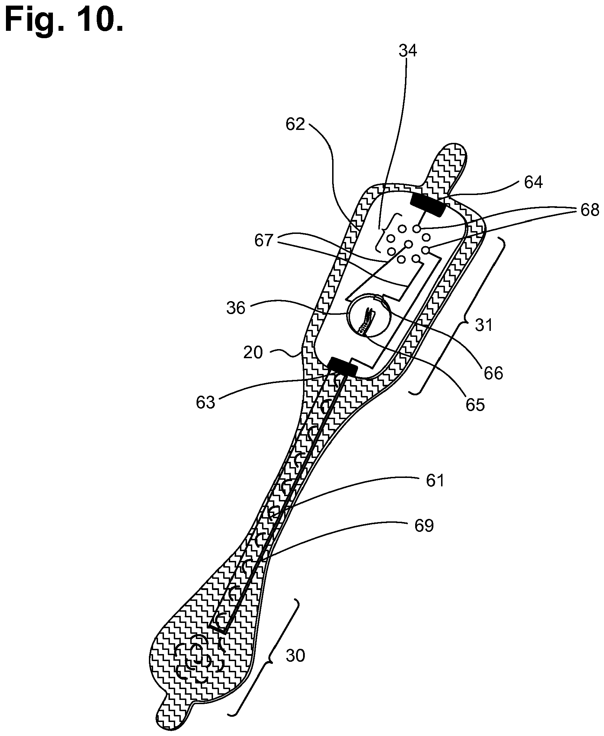

In a still further embodiment, the flexile wires are uninsulated. FIGURE 10 is perspective view of the flexile wire electrode assembly from FIGURE 9, with a layer of insulating material 69 shielding a bare uninsulated distal wire 61 around the midsection on the contact side of the flexible backing. On the contact side of the proximal and distal ends of the flexible backing, only the portions of the flexile wires serving as electrode signal pickups are electrically exposed and the rest of the flexile wire on the contact side outside of the proximal and distal ends are shielded from electrical contact. The bare uninsulated distal wire 61 may be insulated using a layer of plastic, rubber-like polymers, or varnish, or by an additional layer of gauze or adhesive (or non-adhesive) gel. The bare uninsulated wire 61 on the noncontact side of the flexible backing may be insulated or can simply be left uninsulated.

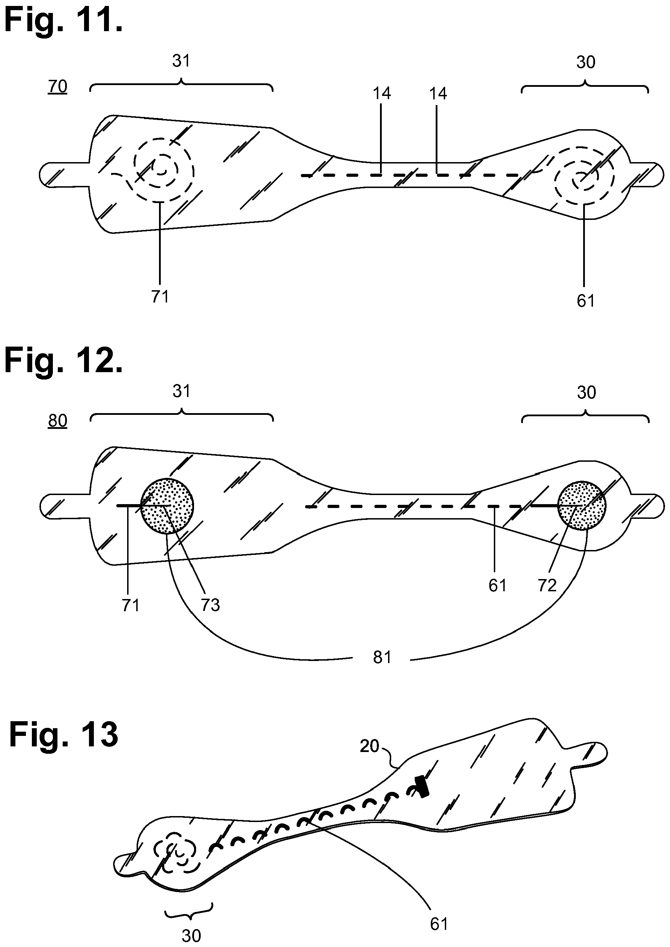

Both end portions of the pair of flexile wires are typically placed uninsulated on the contact surface of the flexible backing 20 to form a pair of electrode signal pickups. FIGURE 11 is a bottom view of the flexile wire electrode assembly as shown in FIGURE 9. When adhered to the skin during use, the uninsulated end portions of the distal wire 61 and the proximal wire 71 enable the monitor recorder 14 to measure dermal electrical potential differentials. At the proximal and distal ends of the flexible backing 20, the uninsulated end portions of the flexile wires may be configured into an appropriate pattern to provide an electrode signal pickup, which would typically be a spiral shape formed by guiding the flexile wire along an inwardly spiraling pattern. The surface area of the electrode pickups can also be variable, such as by selectively removing some or all of the insulation on the contact surface. For example, an electrode signal pickup arranged by sewing insulated flexile wire in a spiral pattern could have a crescent-shaped cutout of uninsulated flexile wire facing towards the signal source.

In a still yet further embodiment, the flexile wires are left freely riding on the contact surfaces on the distal and proximal ends of the flexible backing, rather than being interlaced into the ends of the flexible backing 20. FIGURE 12 is a bottom view of a flexile wire electrode assembly in accordance with a still yet further embodiment. The distal wire 61 is interlaced onto the midsection and extends an exposed end portion 72 onto the distal end 30. The proximal wire 71 extends an exposed end portion 73 onto the proximal end 31. The exposed end portions 72 and 73, not shielded with insulation, are further embedded within an electrically conductive adhesive 81. The adhesive 81 makes contact to skin during use and conducts skin electrical potentials to the monitor recorder 14 (not shown) via the flexile wires. The adhesive 81 can be formed from electrically conductive, non-irritating adhesive, such as hydrocolloid.

The distal wire 61 is interlaced or sewn through the longitudinal midsection of the flexible backing 20 and takes the place of the flexible circuit 32. FIGURE 13 is a perspective view showing the longitudinal midsection of the flexible backing of the electrode assembly from FIGURE 9. Various stitching patterns may be adopted to provide a proper combination of rigidity and flexibility. In simplest form, the distal wire 61 can be manually threaded through a plurality of holes provided at regularly-spaced intervals along an axial path defined between the battery printed circuit board 62 (not shown) and the distal end 30 of the flexible backing 20. The distal wire 61 can be threaded through the plurality of holes by stitching the flexile wire as a single "thread." Other types of stitching patterns or stitching of multiple "threads" could also be used, as well as using a sewing machine or similar device to machine-stitch the distal wire 61 into place, as further described *infra.* Further, the path of the distal wire 61 need not be limited to a straight line from the distal to the proximal end of the flexible backing 20.

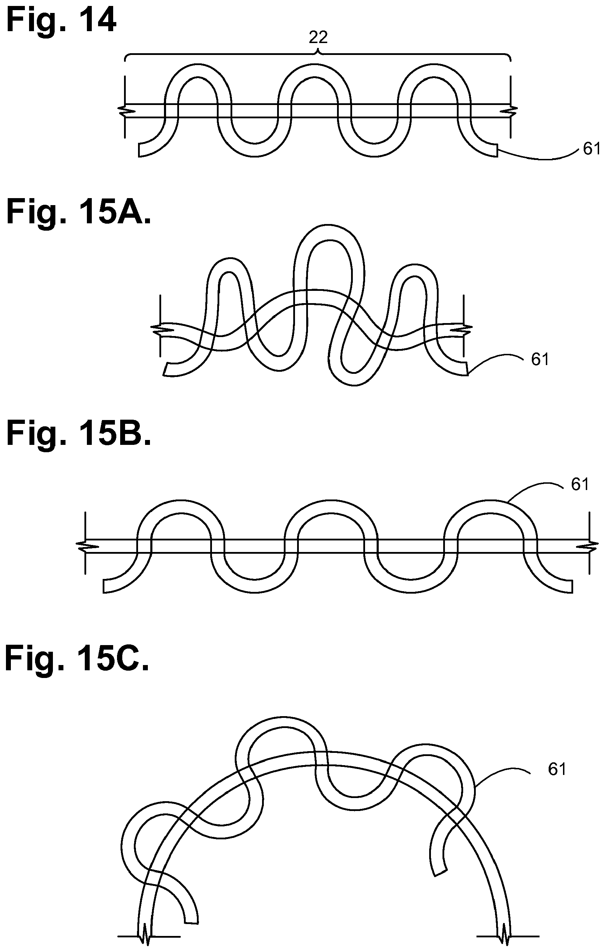

The distal wire 61 is flexile yet still retains a degree of rigidity that is influenced by wire gauge, composition, stranding, insulation, and stitching pattern. For example, rigidity decreases with wire gauge; and a solid core wire tends to be more rigid than a stranded core of the same gauge. The combination of the flexibility and the rigidity of the portion of the distal wire 61 located on or close to the midsection contributes to the overall strength and wearability of the patch. FIGURE 14 is a longitudinal cross-sectional view of the midsection of the flexible backing 20 of the electrode assembly of FIGURE 11. FIGURES 15A-C are the electrode assembly from FIGURE 14 under compressional, tensile, and bending force, respectively. The relative sizes of the distal wire 61 and flexible backing 20 are not to scale and are exaggerated for purposes of illustration.

The interlacing of the distal wire 61 through the narrow longitudinal midsection 22 of the flexible backing 20 bends the distal wire 61 into a line of rounded stitches that alternate top and bottom, which can be advantageous to long term wearability. First, the tension of the rounded stitches reinforces the planar structure of the narrow longitudinal midsection 22 and spreads a dislodging force impacting on one end of the flexible backing 20 to the other end of the flexible backing 20. Second, the rounded stitches leave room for stretching, compressing, bending, and twisting, thus increasing the wearability of the patch extended wear electrode patch 15 by facilitating extension, compression, bending, and twisting of the narrow longitudinal midsection 22 in response to tensile, compressional, bending, and torsional forces.

In a further embodiment, the distal wire and the proximal wire may be stitched or sewn into the flexible backing 20. Depending upon the type of stitching used, the distal or proximal wire may use more than one individual wire. For instance, a conventional sewing machine used to stitch fabrics uses a spool of thread and a bobbin, which are both wound with thread that together allow the creation of various stitching patterns, such as the lockstitch. Other type of stitching patterns are possible. Additionally, where more than one "threads" are used for stitching, the flexile wire may constitute all of the "threads," thereby increasing redundancy of the circuit trace thus formed. Alternatively, just one (or fewer than all) of the threads may be conductive, with the non-conductive threads serving to reinforce the strength of the flexile wire connections and flexible backing 20. The additional threads can be made from line, threads, or fabrics of sufficient mechanical strength and do not need to be conductive; alternatively, the same flexile wires can be employed to serve as the additional threads.

Conventionally, flexible circuits, such as the flexible circuit 32 (shown in FIGURE 4) that connects the distal ECG electrode 38 and proximal ECG electrode 39 (shown in FIGURE 5) to the battery printed circuit board 62 (shown in FIGURE 9), are constructed using subtractive processes. In general, a flexible circuit interconnects electronic components with custom point-to-point circuit traces and is typically constructed by forming the conductive circuit traces on a thin film of insulating polymer. A flexible circuit is not an off-the-shelf component; rather, each flexible circuit is designed with a specific purpose in mind. Changes to a flexible circuit's design will generally require fabricating entirely new flexible circuits, as the physical circuit traces on the polymer film cannot be changed.

Manufacturing a flexible circuit typically requires the use of sophisticated and specialized tools, coupled with environmentally unfriendly processes, including depositing copper on a polyamide core, etching away unwanted copper with inline etching or an acid bath to retain only the desired conductive circuit traces, and applying a coverlay to the resulting flexible circuit. Significant amounts of hazardous waste are generated by these subtractive processes during the fabrication of each flexible circuit. Properly disposing of such hazardous waste is expensive and adds to the costs of the flexible circuit.

In the still further embodiment described *supra* beginning with reference to FIGURE 9, the distal and proximal flexile wires replace the

flexible circuit 32 and enables the electrode assembly to be constructed using additive processes with off-the-shelf, low cost components. The flexile wires serve the triple functions of an electrode signal pickup, electrical circuit trace, and support for structural integrity and malleability of the electrode assembly.

The general manner of constructing the electrode assembly can be applied to other forms of electronic components in which custom point-to-point circuit traces need to be affixed to a gauze or textile backing, as well as backings made from other materials. The circuit traces are replaced by the interlaced or sewn flexile wires, and the ends of each flexile wire are terminated, as appropriate to the application. The flexile wires may, by example, connect two circuit boards, or connect to an electrical terminal, power source, or electrical component. In addition, flexile wires may be used to replace a printed circuit board entirely, with each flexile wire serving as a form of sewn interconnect between two or more discrete components, including resistors, capacitors, transistors, diodes, operational amplifiers (op amps) and other integrated circuits, and other electronic or electromechanical components.

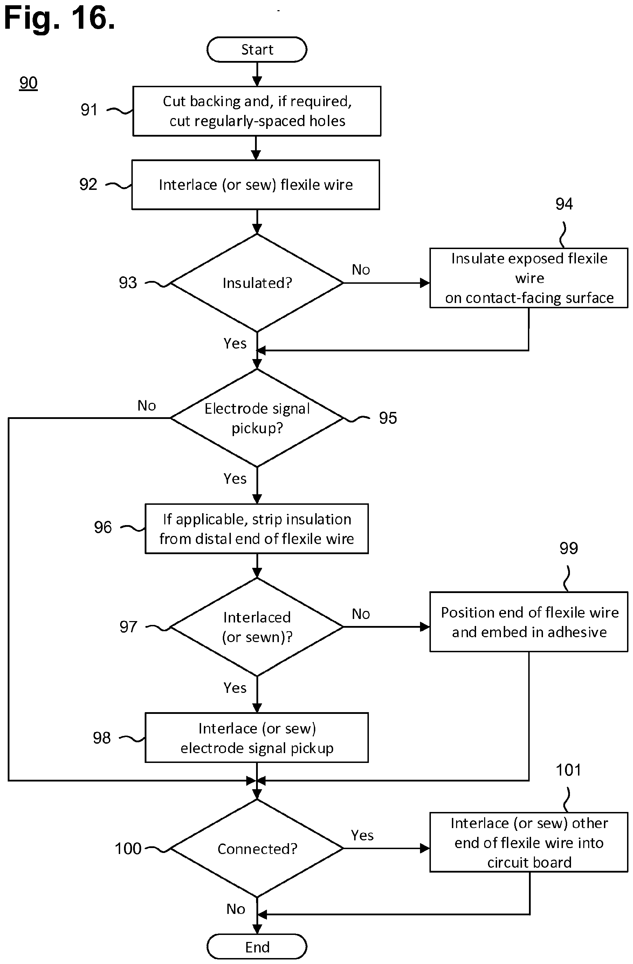

By way of illustration, the flexile wires will be described as terminated for use in an electrode assembly, specifically, as terminated on one end to form an electrode signal pickup and on the other end to connect into a circuit board. Constructing the electrode assembly entails interlacing, including manually threading, or machine sewing the flexile, conductive wire through the flexible backing 20. FIGURE 16 is a flow diagram showing a method 90 for constructing a stress-pliant physiological electrode assembly in accordance with a further embodiment. The method can be performed by a set of industrial machines, including a gauze cutting machine to cut the flexible backing 20 to form; a hole punch to cut a plurality of holes provided at regularly-spaced intervals; a stitching or sewing machine to interleave or sew the flexile wire through the flexible backing 20; a wire stripper or plasma jet to remove insulation from the flexile wire, when applicable; and a glue or adhesive dispenser to embed or coat electrode signal pickup in hydrocolloid gel or equivalent non-irritating adhesive. Other forms or combinations of industrial machines, including a single purpose-built industrial machine, could be used.

As an initial step, a backing is cut to shape and, if required, holes are cut at regularly-spaced intervals along an axial path (step 91) through which the flexile wire will be interlaced. Holes will need to be cut, for instance, if the flexile wire is to be hand-guided through the backing, or where the backing is cut from a material that is difficult to puncture with a threaded needle, such as used by a sewing machine. In one embodiment, the backing is cut from wearable gauze, latex, woven textile, or similar wrap knit or stretchable and wear-safe material, such as a Tricot-type linen; the resulting backing is flexible and yielding. The backing is also cut into an elongated "hourglass"-like shape, when viewed from above, with a pair of cut-outs and a longitudinal midsection that together help minimize interference with and discomfort to breast tissue, particularly in women (and gynecomastic men), such as described *supra* with reference to FIGURE 3. The backing can be cut into other shapes as appropriate to need. In addition, depending upon the application, other materials could be substituted for the backing. For example, neoprene, such as used in wetsuits, could be used where a high degree of elasticity and ruggedness is desired.

The flexile wire is then interlaced or sewn into the backing (step 92). Interlacing can be performed by a machine that guides the flexile wire through the holes previously cut in the material in a crisscrossed, interwoven, or knitted fashion, as well as by hand. The flexile wire can also be guided through the backing without first cutting holes, provided that the weave of the material is sufficiently loose to allow passage of the flexile wire if the flexile wire is otherwise incapable of passing through the backing without the assistance of a needle or other piercing instrument.

Alternatively, the flexile wire could be sewn into the backing by using the flexile wire as "thread" that is stitched into place using a needle or similar implement. If a single flexile wire is employed, the stitching will be a line of rounded stitches that alternate top and bottom, as described *supra;* however, if more than one flexile wire is used, or the stitching pattern requires the use of more than one thread, other forms of conventional machine-stitching patterns could be employed, such as a lockstitch.

Once completed, the interlacing or sewing of the flexile wire into the backing creates an integrated point-to-point electrical path that takes the place of a custom circuit trace using an additive, rather than subtractive, manufacturing process. The flexile wire can be interlaced or sewn along a straight, curved, or arbitrary path. One flexile wire is required per point-to-point circuit trace. The strength and pliability of the flexile wire reinforces the backing and, in the still further embodiment described *supra* beginning with reference to FIGURE 9, facilitates extension, compression, bending, and twisting of the narrow longitudinal midsection 22 in response to tensile, compressional, bending, and torsional forces. Thus, the path of the flexile wire along the backing can be mapped to take advantage of the strength and reinforcing properties of the flexile wire, which, when interlaced or sewn into the backing, help the backing counter the stresses to which the backing will be subjected when deployed.

The flexile wire itself may be insulated or bare (step 93). When one end of the flexile wire is connected to (or forms) an electrode, particularly a dermal physiology electrode that senses electrical potentials on the skin's surface, insulated flexile wire will ordinarily be used, with only a portion of the flexile wire incident to the electrode stripped of insulation. However, bare uninsulated flexile wire could alternatively be used throughout, so long as those portions of the uninsulated flexile wire that are exposed on the contact-facing surface of the backing are insulated and shielded from electrical contact (step 94), such as by applying a layer of plastic, rubber-like polymers, or varnish, or by an additional layer of gauze or adhesive (or non-adhesive) gel over the exposed wire. The uninsulated flexile wire exposed on other surfaces of the backing could also be insulated or simply be left bare.

One end of the flexile wire may be terminated as an electrode signal pickup (step 95). If insulated flexile wire is used, a portion of the end of the flexile wire is stripped of insulation (step 96) using, for instance, a wire stripper or plasma jet. The electrode signal pickup could either be formed by interlacing (or sewing) the flexile wire (step 97) into the backing in the shape of the desired electrode (step 98) or positioned over the contact-facing area of the backing designated to serve as an electrode signal pickup and embedded within an electrically conductive adhesive (step 99). In a yet further embodiment, the flexile wire could be terminated as a connection to a discrete electrode, such as by sewing an uninsulated portion of the end of the electrode wire into the discrete electrode to thereby establish an electrical contact and affix the discrete electrode to the backing. The Universal ECG EKG electrode, manufactured by Bio Protech Inc., Tustin, CA, is one example of a discrete electrode.

Finally, the other end of the flexile wire may be terminated as a connection to a circuit board (step 100). The flexile wire can be interlaced or sewn onto the circuit board, for instance, by back stitching the flexile wire back and forth across the edge of the circuit board to thereby establish an electrical contact and affix the discrete electrode to the backing.

In a further embodiment, flexile wire can be used to replace all or part of a printed circuit board, such as battery printed circuit board 62 used in constructing a stress-pliant physiological electrode assembly, as described *supra,* or for any other application that requires interconnection of electrical or electro mechanical components on a physical substrate or backing. Flexile wire in place of conductive circuit traces can work especially well with simple circuit board layouts, where ample space between components and relatively uncomplicated layouts are amenable to stitched-in interconnections. In addition, the use of flexile wire can simplify circuit layout design in multilayer circuits, as insulated flexile wires can be run across each other in situations that would otherwise require the use of a multilayer printed circuit board or similar solution.

Through such use of flexile wire, a printed circuit board can be omitted in whole or in part. Interconnects between and connections to the electronic and electro mechanical components formerly placed on the printed circuit board can instead be sewn from flexile wire. For instance, the battery printed circuit board 62 can be replaced by flexile wire interconnects that connect the electrodes to a sewn set of electrical pads formed by over-stitching the flexile wire into electrical contact surfaces of sufficient size to interface with a monitor recorder 14 when fitted into the non-conductive receptacle 25. Likewise, the spring 65 and clasp 66 can be sewn in place using flexile wire to hold a battery in place with flexile wire interconnects connecting the battery to a sewn set of electrical pads formed by over-stitching the flexile wire into electrical contact surfaces of sufficient size to interface with a monitor recorder 14 when fitted into the non-conductive receptacle 25. Still other approaches to replacing printed circuit boards with flexile wire interconnects are possible.

The resultant stress-pliant physiological electrode assembly may be electrically coupled to a broad range of physiological monitors not limited to electrocardiographic measurement. The foregoing method of constructing a stress-pliant electrode assembly is adaptable to manufacturing other forms of dermal electrodes, including electrodes for electrocardiography, electroencephalography, and skin conductance measurements. Further, by adjusting the number of electrodes, the distances among the electrode signal pickups, and the thickness of the flexile wire, the method can be adapted to manufacturing at low cost an electrode assembly that is lightweight and resistant to tensile, compressional and torsional forces, thus contributing to long-term wear and versatility.

While the invention has been particularly shown and described as referenced to the embodiments thereof, those skilled in the art will understand that the foregoing and other changes in form and detail may be made therein without departing from the spirit and scope.

The present divisional application should be taken to extend to include all of the subject matter of the parent application, the entire contents of which are incorporated by reference and more particularly the claims of the parent application as filed which are reproduced below as a series of numbered statements.
1. An extended wear electrocardiography patch (15) using interlaced wire electrodes, comprising:
   a flexible backing (20) formed of an elongated strip (21) of stretchable material (44) with a narrow longitudinal midsection (23) evenly tapering inward from distal end (30) and a proximal end (31), the elongated strip (21) adherable only to a contact surface defined on each of the ends (30, 31);
   a pair of flexile wires (61, 71);
   a pair of electrodes, one such electrode comprising one of the flexile wires (61) interlaced into the distal end (30) of the elongated strip (21) and continuing back along an axial path through the midsection (23), another such electrode comprising the other of the flexile wires (71) interlaced into the proximal end (31) of the elongated strip (21), each of the electrodes further comprising an electrically conductive area (72, 73) only exposed on the contact surface;
   a circuit board (62) affixed on the proximal end (31) of the elongated strip (21), comprising a battery compartment (36) operable to hold a battery for powering an electrocardiography monitor (14), and an electrical pad (34) operable to electrically couple the electrocardiography monitor (14) with the pair of the electrodes; and
   a non-conductive receptacle (25) securely adhered on the one end (31) of the elongated strip (21) opposite the contact surface and formed to removably receive the electrocardiography monitor (14), the non-conductive receptacle (25) comprising electrode terminals aligned to electrically interface the pair of the flexile wires (61, 71) to the electrocardiography monitor..
2. An electrode patch (15) according to Statement 1, further comprising:
   one of the electrocardiographic electrodes being disposed for being adhered to the region overlying the lower sternum on a patient's chest; and
   an other of the electrocardiographic electrodes being disposed for being adhered to the region near the manubrium or upper sternum upwards on the patient's chest oriented centrally (in the midline (16)) along the sternum (13) from the one electrocardiographic electrode.
3. An electrode patch (15) according to Statement 1, further comprising:
   an upper part of the elongated strip (21) defined on one end (31) of the flexible backing (20) and sized to be affixed to the bottom surface of the non-conductive receptacle (25), one of the electrocardiographic electrodes being affixed to and conductively exposed on the contact surface of the upper part; and
   a lower part of the elongated strip (21) defined on another end (30) of the flexible backing (20), another of the electrocardiographic electrodes being affixed to and conductively exposed on the contact surface of the lower part,
   wherein the lower part of the elongated strip (21) is provided for being adhered to the region overlying the lower sternum on a patient's chest with the narrow longitudinal midsection (23) oriented centrally (in the midline (16)) along the sternum 13 and the upper part of the elongated strip (21) oriented upwards towards the manubrium.
4. An electrode patch (15) according to Statement 1, further comprising:
   a non-irritating adhesive dressing (43) at least partially coated on each end (30, 31) of the elongated strip (21) on the contact surface.
5. An electrode patch (15) according to Statement 1, further comprising:
   at least one pattern of interlaced flexile wires (61, 71) on the ends (30, 31) of the elongated strip (21) selected from the group of a circle, a square, a triangle, and an arbitrary shape.
6. An electrode patch (15) according to Statement 1, further comprising:
   a plurality of holes arranged at regularly spaced intervals along an axial path defined between the circuit board (62) and the distal end (30) of the flexible backing (20), through which one of the flexible wires (61) is threaded.
7. An electrode patch (15) according to Statement 1, further comprising:
   a layer of insulation material (69) covering at least part of a surface of the flexile wires (61, 71).
8. An electrode patch (15) according to Statement 1, wherein the flexile wires (61, 71) comprise at least one of copper, aluminum, silver, and other metals.
9. An electrode patch (15) according to Statement 1, wherein a core of the flexile wires (61, 71) comprises at least one of solid, stranded, and braided form.
10. An electrode patch (15) according to Statement 1, further comprising:
   the battery compartment (36) provided in the non-conductive receptacle (25) and comprising a pair of battery leads (67) electrically coupleable to a battery; and
   the non-conductive receptacle (25) further comprising power terminals aligned to electrically interface the pair of battery leads (67) to the electrocardiography monitor (14).
11. An extended wear electrocardiography patch using non-metal electrodes (15), comprising:
   a flexible backing (20) formed of an elongated strip (21) of stretchable material (44) with a narrow longitudinal midsection (23) evenly tapering inward from a distal end (30) and a proximal end (31), the elongated strip (21) adherable only to a contact surface defined on each of the ends (30, 31);
   a pair of flexile wires (61, 71);
   a pair of electrodes, one such electrode comprising one of the flexile wires (61) positioned on the distal end (30) of the elongated strip (21) on the contact surface and continuing back along an axial path through the narrow longitudinal midsection (23), another such electrode comprising the other of the flexile wires (71) positioned on the proximal end (31) of the elongated strip (21) on the contact surface, each of the electrodes being imbedded in an electrically conductive adhesive (81);
   a circuit board (62) affixed on the proximal end (31) of the elongated strip (21), comprising a battery compartment (36) operable to hold a battery for powering an electrocardiography monitor (14), and an electrical pad (34) operable to electrically couple the electrocardiography monitor (14) with the pair of the electrodes; and
   a non-conductive receptacle (25) securely adhered on the one end (31) of the elongated strip (21) opposite the contact surface and formed to removably receive the electrocardiography monitor (14), the non-conductive receptacle (25) comprising electrode terminals aligned to electrically interface the pair of the flexile wires (61, 71) to the electrocardiography monitor (14).
12. An electrode patch (15) according to Statement 11, further comprising:
   one of the electrocardiographic electrodes being disposed for being adhered to the region overlying the lower sternum on a patient's chest; and
   an other of the electrocardiographic electrodes being disposed for being adhered to the region near the manubrium upwards on the patient's chest oriented centrally (in the midline (16)) along the sternum (13) from the one electrocardiographic electrode.
13. An electrode patch (15) according to Statement 11, further comprising:
   an upper part of the elongated strip (21) defined on one end (31) of the flexible backing (20) and sized to be affixed to the bottom surface of the non-conductive receptacle (25), one of the electrocardiographic electrodes being affixed to and conductively exposed on the contact surface of the upper part; and
   a lower part of the elongated strip (21) defined on another end (30) of the flexible backing (20), another of the electrocardiographic electrodes being affixed to and conductively exposed on the contact surface of the lower part,
   wherein the lower part of the elongated strip (21) is provided for being adhered to the region overlying the lower sternum on a patient's chest with the narrow longitudinal midsection (23) oriented centrally (in the midline (16)) along the sternum (13) and the upper part of the elongated strip (21) oriented upwards towards the manubrium.
14. An electrode patch (15) according to Statement 11, further comprising:
   a non-irritating adhesive dressing (43) at least partially coated on each end (30, 31) of the elongated strip (21) on the contact surface.
15. An electrode patch (15) according to Statement 14, further comprising:
   a contoured surface comprised on the non-irritating adhesive dressing (43) on at least one end (30, 31) of the elongated strip (21).
16. An electrode patch (15) according to Statement 11, further comprising:
   at least one pattern of interlaced flexile wires (61, 71) on the ends (30, 31) of the elongated strip (21) selected from the group of a circle, a square, a triangle, and an arbitrary shape.
17. An electrode patch (15) according to Statement 11, further comprising:
   a plurality of holes arranged at regularly spaced intervals along an axial path defined between the circuit board (62) and the distal end (30) of the flexible backing (20), through which one of the flexible wires (61) is threaded.
18. An electrode patch (15) according to Statement 11, further comprising:
   a layer of insulation material (69) covering at least part of a surface of the flexile wires (61, 71).
19. An electrode patch (15) according to Statement 11, further comprising:
   a moisture-resistant seal (35) formed on the non-conductive receptacle (25) circumferentially about the electrode terminals and adapted to substantially protect the electrocardiography monitor (14) against moisture intrusion.
20. An electrode patch (15) according to Statement 11, further comprising:
   the battery compartment (36) provided in the non-conductive receptacle (25) and comprising a pair of battery leads (67) electrically coupleable to a battery; and
   the non-conductive receptacle (25) further comprising power terminals aligned to electrically interface the pair of battery leads (67) to the electrocardiography monitor (14).
21. An extended wear electrocardiography patch (15), comprising:
   a flexible backing (20) formed of an elongated strip (21) of stretchable material (44) with a narrow longitudinal midsection (23) evenly tapering inward from both ends (30, 31), the elongated strip (21) adherable only on a contact surface defined on each of the ends (30, 31), and operable to serve as a crimp relief to facilitate compression of the narrow longitudinal midsection (23) in response to compressional and torsional forces;
   a pair of electrocardiographic electrodes (38, 39) respectively affixed to and conductively exposed on the contact surface of each end (30, 31) of the elongated strip (21);
   a flexible circuit (32) affixed on each end (30, 31) to the elongated strip (21) and comprising a pair of circuit traces (33, 37) each originating within one of the ends of the elongated strip (21), respectively, and electrically coupled to one of the electrocardiographic electrodes (38, 39), respectively;
   a laterally-extendable strain relief (40) defined in the flexible circuit (32) and operable to facilitate extension and rotation of the flexible circuit (32) in response to tensile and torsional forces; and
   a non-conductive receptacle (25) securely adhered on one end (31) of the elongated strip (21) opposite the contact surface and operable to removably receive an electrocardiography monitor (14), the non-conductive receptacle (25) comprising aligned to electrically interface the pair of the circuit traces (33, 37) to the electrocardiography monitor (14).
22. An electrocardiography patch (15) according to Statement 21, further comprising:
   one of the electrocardiographic electrodes (38) being disposed for being adhered to a region overlying the lower sternum on a patient's chest; and
   an other of the electrocardiographic electrodes (39) being disposed for being adhered to the region near the manubrium on the patient's chest oriented centrally (in the midline (16)) along the sternum (13) upwards from the one electrocardiographic electrode (38).
23. An electrocardiography patch according to Statement 21, further comprising:
   an upper part of the elongated strip (21) defined on one end (31) of the flexible backing (20) and sized to be affixed to the bottom surface of the non-conductive receptacle (25), the other of the electrocardiographic electrodes (39) being affixed to and conductively exposed on the contact surface of the upper part; and
   a lower part of the elongated strip (21) defined on another end (30) of the flexible backing (20), the one of the electrocardiographic electrodes (38) being affixed to and conductively exposed on the contact surface of the lower part,
   wherein the lower part of the elongated strip (21) is provided for being adhered to the region overlying the lower sternum on a patient's chest with the narrow longitudinal midsection (23) oriented centrally (in the midline (16)) along the sternum 13 and the upper part of the elongated strip (21) oriented upwards towards the manubrium.
24. An electrocardiography patch (15) according to Statement 21, further comprising:
   a non-irritating adhesive dressing (43) at least partially coated on each end of the elongated strip (21) on the contact surface.
25. An electrocardiography patch (15) according to Statement 21, further comprising:
   a pair of strain relief cutouts (41) comprised in the strain relief (40) that each face towards the other strain relief cutout (41), each strain relief cutout (41) partially extending transversely from and continuing longitudinally to a side of the flexible circuit (32) different than the other strain relief cutout (41), one of the pair of the circuit traces (33, 37) following a path formed on the flexible circuit (32) between the pair of the strain relief cutouts (41).
26. An electrocardiography patch (15) according to Statement 25, wherein the pair of the strain relief cutouts (41) define an 'S'-shaped pattern in the flexible circuit (32) when viewed from above.
27. An electrocardiography patch (15) to Statement 21, wherein the narrow longitudinal midsection (23) defines an hourglass-shaped pattern in the flexible backing (20) when viewed from above conformal to fit comfortably within the intermammary cleft of a patient's pair of breasts.
28. An electrocardiography patch (15) according to Statement 21, further comprising:
   a battery compartment (36) provided in the non-conductive receptacle and comprising a pair of battery leads electrically coupleable to a battery; and
   the non-conductive receptacle (25) further comprising power terminals aligned to electrically interface the pair of battery leads to the electrocardiography monitor (14).
29. An electrocardiography patch (15) according to Statement 21, further comprising:
   a moisture-resistant seal (35) formed on the non-conductive receptacle (25) circumferentially about the electrode terminals and adapted to substantially protect the electrocardiography monitor (14) against moisture intrusion.
30. An electrocardiography patch (15) according to Statement 1, further comprising:
   a contoured surface comprised on the non-irritating adhesive dressing (43) on at least one end of the elongated strip (21).
31. An electrocardiography patch (15) according to Statement 21, further comprising:
   a plurality of fasteners comprised on the non-conductive receptacle (25) and oriented to securely receive and to hold captive the electrocardiography monitor (14) into the non-conductive receptacle (25).
32. An electrocardiography patch according to Statement 21, further comprising at least one of:
   rounded edges defined about a bottom surface of the non-conductive receptacle (25);
   a gel well (46) circumferentially defined about at least one of the electrocardiographic electrodes (38, 39) on the contact surface of the end (30, 31) of the flexible backing (20) on which the at least one electrocardiographic electrode (38, 39) is affixed; and
   placement and removal tabs (24) comprised as part of the flexible backing (20) and extending longitudinally beyond at least one end (30, 31) of the flexible backing (20).
33. An extended wear hourglass-shaped electrocardiography patch (15), comprising:
   a flexible backing (20) formed of an elongated strip (21) of stretchable material (44) with a narrow longitudinal midsection (23) evenly tapering inward from both ends (30, 31) into an hourglass-shaped pattern when viewed from above;
   a contact surface of the flexible backing (20) comprising a non-irritating adhesive dressing (43) at least partially coated on each end and lacking the non-irritating adhesive dressing (43) on the narrow longitudinal midsection (23);
   a pair of electrocardiographic electrodes (38, 39) respectively affixed to and conductively exposed on the contact surface of each end of the flexible backing (20);
   a flexible circuit (32) affixed to each end (30, 31) of the flexible backing (20), comprising:
      a pair of circuit traces (33, 37) each originating within one of the ends (30, 31) of the flexible backing (20), respectively, each circuit trace (33, 37) electrically coupled to one of the electrocardiographic electrodes (38, 39), respectively; and
      a pair of strain relief cutouts (41) that each face towards the other strain relief cutout (41), each strain relief cutout (41) partially extending transversely from and continuing longitudinally to a side of the flexible circuit (32) different than the other strain relief cutout (41), one of the pair of the circuit traces (33, 37) following a path formed on the flexible circuit (32) between the pair of the strain relief cutouts (41); and
   a non-conductive receptacle (25) securely adhered on the one end (31) of the flexible backing (20) opposite the contact surface and operable to removably receive an electrocardiography monitor (14).
34. An electrocardiography patch (15) according to Statement 33, wherein one of the electrocardiographic electrodes (38) is disposed for being adhered to a region overlying the lower sternum on a patient's chest and the other of the electrocardiographic electrodes (39) is disposed for being adhered to the region in near the manubrium and oriented centrally (in the midline (16)) along the sternum (13) upwards from the one electrocardiographic electrode (38).
35. An electrocardiography patch (15) according to Statement 33, further comprising:
   an upper part of the hourglass-shaped pattern sized to seat the non-conductive receptacle (25) on an outward-facing surface of the flexible backing (20); and
   a lower part of the hourglass-shaped pattern comprising a size smaller than the upper part of the hourglass-shaped pattern.
36. An electrocardiography patch (15) according to Statement 35, wherein the lower part is provided for being adhered to the region overlying the lower sternum on a patient's chest with the narrow longitudinal midsection (23) oriented centrally (in the midline (16)) along the sternum (13) and the upper part oriented upwards towards the manubrium.
37. An electrocardiography patch (15) according to Statement 33, further comprising:
   the non-conductive receptacle (25) comprising electrode terminals aligned to electrically interface the electrocardiographic electrodes (38, 39) via the pair of the circuit traces (33, 37) to the electrocardiography monitor (14).
38. An electrocardiography patch (15) according to Statement 33, further comprising:
   a pair of concave cut-outs (22) defined along the longitudinal edges of the elongated strip (21), each concave cut-out (22) contoured conformal to substantially clear an intermammary cleft.
39. An electrocardiography patch (15) according to Statement 33, further comprising:
   a battery comprised in the non-conductive receptacle (25) and disposed to provide power to the electrocardiography monitor (14).
40. An electrocardiography patch (15) according to Statement 33, wherein the stretchable material (44) comprises at least one of wearable gauze, wearable latex, wrap knit fabric, and Tricot linen.
41. A method (90) for constructing a stress-pliant physiological electrode assembly, comprising:
   forming an electrode backing (20) comprising of stretchable woven textile material (44) compatible to contact the skin on at least one surface;
   obtaining a length of flexile wire (61) with a cross-sectional girth greater than the interstices formed between individual threads comprised in the stretchable woven textile material (44);
   defining an electrical contact area on the at least one surface area and providing an electrically-contacting end (72) of the flexile wire (61) substantially within the electrical contact area; and
   interlacing (92) the flexile wire (61) through the stretchable woven textile material (44) from the electrical contact area to a surface of the stretchable woven textile material (44) opposite to the electrical contact area and oriented in a manner operable to receive an electrical connection.
42. A method (90) according to Statement 41, further comprising:
   interlacing (98) the electrically-contacting end (72) of the flexile wire (61) through substantially the electrical contact area, with the electrically-contacting end (72) being disposed for electrode signal pickup.
43. A method (90) according to Statement 42, further comprising:
   interlacing (98) the flexile wire (61) via a pattern that facilitates the electrical signal pickup.
44. A method (90) according to Statement 42, wherein the flexile wire (61) comprises a conductor core and at least one layer of insulator (69) surrounding the conductor core.
45. A method (90) according to Statement 44, further comprising:
   removing (96) at least a portion of the layer of the insulator (69) surrounding the electrically-contacting end (72) of the flexile wire (61) substantially within the electrical contact area, to be performed before, during, or after the process of interlacing the electrically-contacting end (72) of the flexile wire (61) through substantially the electrical contact area.
46. A method (90) according to Statement 45, wherein the removal (96) of the insulator is performed by at least one of the processes of heating, laser, cutting, abrasion, and stripping.
47. A method (90) according to Statement 41, further comprising:
   defining on the electrode backing (20) a distal end (30), a proximal end (31), and a midsection between the two ends, wherein the flexile wire (61) is interlaced substantially through the midsection, and the electrical contact area is within the distal end (30);
   providing a further flexile wire (71);
   defining a further electrical contact area on the proximal end (31) and providing a further electrically-contacting end (73) of the further flexile (71) wire substantially within the further electrical contact area; and
   interlacing the further flexile wire (71) through the stretchable woven textile material (44) from the further electrical contact area to the surface of the stretchable woven textile material (44) opposite to the electrical contact area and oriented in a manner operable to receive an electrical connection.
48. A method (90) according to Statement 47, further comprising:
   processing the midsection into a narrow isthmus tapering inwards from both sides of the midsection, to provide for the distal end electrode being disposed for being adhered to the region overlying the lower sternum on a patient's chest, and to further provide for the proximal electrode being disposed for being adhered to the region near the manubrium upwards on the patient's chest oriented centrally (in the midline (16)) along the sternum (13).
49. A method (90) according to Statement 47, further comprising:
   affixing a circuit board (62) onto the surface of the stretchable woven textile material (44) opposite to the electrical contact area, comprising a battery compartment (36) operable to hold a battery for powering a physiological monitor (14), and an electrical pad (34) operable to electrically couple the physiological monitor (14) to the flexile wire (61) and the further flexile wire (71); and
   adhering a non-conductive receptacle (25) securely on top of the circuit board (62) and formed to removably receive the physiological monitor (14), the non-conductive receptacle (25) comprising electrode terminals aligned to electrically interface the flexile wire (61) and the further flexile wire (71) to the physiological monitor (14).
50. A method (90) according to Statement 49, wherein the physiological monitor (14) comprises an electrocardiography monitor.
51. A method (90) according to Statement 41, further comprising:
   affixing an electrically conductive adhesive (81) at least partially onto the electrically-contacting end (72) of the flexile wire (61) substantially within the electrical contact area, with the electrically conductive adhesive (81) being disposed for electrode signal pickup and affixing the electrically-contacting end (72) to the electrical contact area.
52. A method (90) according to Statement 41, wherein the electrically conductive adhesive (81) comprises hydrocolloid.
53. A method (90) according to Statement 51, wherein the flexile wire (61) comprises a conductor core and at least one layer of insulator surrounding the conductor core.
54. A method (90) according to Statement 53, further comprising:
   removing (96) at least a portion of the layer of the insulator (69) surrounding the electrically-contacting end (72) of the flexile wire (61) substantially within the electrical contact area, to be performed before or during the process of affixing the electrically conductive adhesive at least partially onto the electrically-contacting end (72) of the flexile wire (61).
55. A method (90) according to Statement 54, wherein the removal (96) of the insulator (69) is performed by at least one of the processes of heating, laser, cutting, abrasion, and stripping.
56. A method (90) according to Statement 41, further comprising:
   covering the interlaced flexile conductive wire (61) with at least one layer of an insulation material (69).
57. A method (90) according to Statement 41, further comprising:
   adhering at least partially to the at least one surface of the electrode backing (20) a non-irritating adhesive dressing (43).
58. A method (90) according to Statement 41, wherein the pair of the flexile conductive wires (61, 71) comprises at least one of the group of copper, aluminum, silver, and silver compounds.
59. A method (90) according to Statement 41, wherein interlacing (92) the flexile wire (61) is performed through stitching, sewing, or printing.
60. A method (90) according to Statement 41, wherein the flexile wire (61) is chosen from a group of solid, stranded, and braided wires.

## Claims

1. An extended wear electrocardiography patch using non-metal electrodes (15), comprising:
a flexible backing (20) formed of an elongated strip (21) of stretchable material (44) with a narrow longitudinal midsection (23) evenly tapering inward from a distal end (30) and a proximal end (31), the elongated strip (21) adherable only to a contact surface defined on each of the ends (30, 31);
a pair of flexile wires (61, 71);
a pair of electrodes, one such electrode comprising one of the flexile wires (61) positioned on the distal end (30) of the elongated strip (21) on the contact surface and continuing back along an axial path through the narrow longitudinal midsection (23), another such electrode comprising the other of the flexile wires (71) positioned on the proximal end (31) of the elongated strip (21) on the contact surface, each of the electrodes being imbedded in an electrically conductive adhesive (81);
a circuit board (62) affixed on the proximal end (31) of the elongated strip (21), comprising a battery compartment (36) operable to hold a battery for powering an electrocardiography monitor (14), and an electrical pad (34) operable to electrically couple the electrocardiography monitor (14) with the pair of the electrodes; and
a non-conductive receptacle (25) securely adhered on the one end (31) of the elongated strip (21) opposite the contact surface and formed to removably receive the electrocardiography monitor (14), the non-conductive receptacle (25) comprising electrode terminals aligned to electrically interface the pair of the flexile wires (61, 71) to the electrocardiography monitor (14).

2. An electrode patch (15) according to Claim 1, further comprising:
one of the electrocardiographic electrodes being disposed for being adhered to the region overlying the lower sternum on a patient's chest; and
an other of the electrocardiographic electrodes being disposed for being adhered to the region near the manubrium upwards on the patient's chest oriented centrally (in the midline (16)) along the sternum (13) from the one electrocardiographic electrode.

3. An electrode patch (15) according to Claim 1, further comprising:
an upper part of the elongated strip (21) defined on one end (31) of the flexible backing (20) and sized to be affixed to the bottom surface of the non-conductive receptacle (25), one of the electrocardiographic electrodes being affixed to and conductively exposed on the contact surface of the upper part; and
a lower part of the elongated strip (21) defined on another end (30) of the flexible backing (20), another of the electrocardiographic electrodes being affixed to and conductively exposed on the contact surface of the lower part,
wherein the lower part of the elongated strip (21) is provided for being adhered to the region overlying the lower sternum on a patient's chest with the narrow longitudinal midsection (23) oriented centrally (in the midline (16)) along the sternum (13) and the upper part of the elongated strip (21) oriented upwards towards the manubrium.

4. An electrode patch (15) according to Claim 1, further comprising:
a non-irritating adhesive dressing (43) at least partially coated on each end (30, 31) of the elongated strip (21) on the contact surface.

5. An electrode patch (15) according to Claim 4, further comprising:
a contoured surface comprised on the non-irritating adhesive dressing (43) on at least one end (30, 31) of the elongated strip (21).

6. An electrode patch (15) according to Claim 1, further comprising:
at least one pattern of interlaced flexile wires (61, 71) on the ends (30, 31) of the elongated strip (21) selected from the group of a circle, a square, a triangle, and an arbitrary shape.

7. An electrode patch (15) according to Claim 1, further comprising:
a plurality of holes arranged at regularly spaced intervals along an axial path defined between the circuit board (62) and the distal end (30) of the flexible backing (20), through which one of the flexible wires (61) is threaded.

8. An electrode patch (15) according to Claim 1, further comprising:
a layer of insulation material (69) covering at least part of a surface of the flexile wires (61, 71).

9. An electrode patch (15) according to Claim 1, further comprising:
a moisture-resistant seal (35) formed on the non-conductive receptacle (25) circumferentially about the electrode terminals and adapted to substantially protect the electrocardiography monitor (14) against moisture intrusion.

10. An electrode patch (15) according to Claim 1, further comprising:
the battery compartment (36) provided in the non-conductive receptacle (25) and comprising a pair of battery leads (67) electrically coupleable to a battery; and
the non-conductive receptacle (25) further comprising power terminals aligned to electrically interface the pair of battery leads (67) to the electrocardiography monitor (14).

11. An electrode patch (15) according to Claim 1, wherein the flexile wires (61, 71) comprise at least one of copper, aluminum, silver, and other metals.

12. An electrode patch (15) according to Claim ,1 wherein a core of the flexile wires (61, 71) comprises at least one of solid, stranded, and braided form.

13. An electrode patch (15) according to Claim 1, wherein ends of the flexile wires (61, 71) are uninsulated and measure dermal electrical potential differentials.

14. An electrode patch (15) according to Claim 13, wherein the ends of the flexile wires (61, 71) are configured into a pattern to provide an electrical signal pickup.

15. An electrode patch (15) according to Claim 14, wherein the pattern of the ends of the flexile wires (61, 71) comprises a spiral.
